# EUROPÄISCHE PATENTSCHRIFT

(11) **EP 3 661 472 B1**
(45) Veröffentlichungstag und Bekanntmachung des Hinweises auf die Patenterteilung: **25.09.2024**
(21) Anmeldenummer: 18762228.7
(22) Anmeldetag: 02.08.2018
(51) Int. Cl.: A61G 13/12, A61G 13/00, A61F 5/042, A61B 90/14

(54) **SCHNELL VERSTELLBARES KOMPAKTES ZUGSPINDELAGGREGAT MIT SELBSTHEMMUNG ZUM EINSATZ BEI CHIRURGISCHEN EINGRIFFEN**
QUICKLY ADJUSTABLE COMPACT SPINDLE TRACTION MECHANISM WITH SELF-LOCKING FOR USE IN SURGICAL INTERVENTIONS
APPAREIL À TIGE DE TRACTION, COMPACT, RÉGLABLE RAPIDEMENT, AUTOBLOQUANT, DESTINÉ À ÊTRE UTILISÉ LORS D'INTERVENTIONS CHIRURGICALES

(30) Priorität: 04.08.2017 DE 102017117767
(43) Veröffentlichungstag der Anmeldung: 10.06.2020
(73) Patentinhaber: Maquet GmbH, 76437 Rastatt (DE)
(72) Erfinder: KOCH, Guido, 76149 Karlsruhe (DE)
(74) Vertreter: Zacco GmbH
(86) Internationale Anmeldenummer: PCT/EP2018/071067
(87) Internationale Veröffentlichungsnummer: WO 2019/025570

(56) Entgegenhaltungen:
- EP-A2- 2 982 880
- DE-A1- 102013 202 700
- DE-U1- 202009 003 314
- US-A- 4 802 464
- US-A1- 2007 251 011
- US-A1- 2010 282 009

## Beschreibung

### Hintergrund der Offenbarung

Die vorliegende Offenbarung betrifft ein Zugspindelaggregat, welches beispielsweise bei einem Extensionsholm an einem Operationstisch eingesetzt werden kann.

Derartige Extensionsholme umfassen üblicherweise einen Holm, an dem mittels einer verschiebbaren Strebe oder dergleichen unter Zwischenschaltung eines Zugspindelaggregats eine Halterung für einen Patientenfuß bewegbar angebracht ist. Nach dem Fixieren der Strebe kann es für einige chirurgische Anwendungen gewünscht sein, die Halterung und den Patientenfuß mittels des Zugspindelaggregats weiter in Richtung einer Längsachse des Patientenbeins zu bewegen, um beispielsweise ein Hüftgelenk des Patienten auseinander zu ziehen. In der Regel wird diese lineare Verstellbewegung über einen Gewindespindeltrieb des Zugspindelaggregats realisiert, den der Anwender über eine Handkurbel bedienen kann. Hierbei wirken relativ hohe axiale Kräfte auf das Zugspindelaggregat, und es ist wichtig, dass eine einmal eingestellte axiale Position des Patientenfußes beibehalten wird, d.h. dass das Zugspindelaggregat selbsthemmend ist. Die Selbsthemmung kann dadurch erreicht werden, dass beim Zugspindelaggregat eine Spindel eingesetzt wird, welche eine relativ geringe Steigung hat. Selbsthemmung liegt immer dann vor, wenn die Reibkraft abhängig zur anliegenden Axialkraft ist und das zugehörige Reibmoment immer größer ist als das durch die Axialkraft erzeugte Drehmoment.

Die geringe Steigung bedeutet aber eine hohe Anzahl an Spindelumdrehungen, um den erforderlichen Weg zu verfahren. Dies ist für den Anwender sehr mühsam. Wenn bei gleichem Spindeldurchmesser eine Spindel mit größerer Steigung verwendet wird, bei der weniger Spindelumdrehungen für einen gegebenen Verstellweg erforderlich sind, ist die Zugspindel jedoch nicht mehr selbsthemmend, da durch den größeren Steigungswinkel die Reibungskräfte zwischen Spindel und Spindelmutter nicht mehr ausreichend sind, um eine selbsttätige Drehung der Zugspindel bei axial einwirkenden Kräften zu verhindern. Die Selbsthemmung könnte trotz größerer Steigung dadurch erreicht werden, dass der Durchmesser der Spindel erhöht wird. Dadurch würde das Zugspindelaggregat aber unerwünscht schwer und sperrig.

Vom Anwender wird es z.B. als angenehm empfunden, wenn pro Kurbelumdrehung 10-15mm Hub ausgeführt werden. Eine Gewindespindel mit dieser Steigung von 10-15mm müsste aber einen Durchmesser von mehr als 40mm haben, um selbsthemmend zu sein. Dies würde das Zugspindelaggregat in unakzeptabler Weise schwer und groß werden lassen. Für die auftretenden Lasten wäre dies auch überdimensioniert.

EP 2 982 880 A2 offenbart ein selbsthemmendes Zugspindelaggregat gemäß dem Oberbegriff des Anspruchs 1.

US 2010/282009 A1 beschreibt ein selbsthemmendes Zugspindelaggregat mit Spindel, Spindelmutter, Schlitten, Antriebseinrichtung und Getriebe.

### Überblick über die Offenbarung

Es ist daher eine Aufgabe der vorliegenden Offenbarung, ein Zugspindelaggregat bereitzustellen, welches sich schnell verfahren lässt und dabei dennoch selbsthemmend und kompakt ist.

Die voranstehend genannte Aufgabe wird durch ein selbsthemmendes Zugspindelaggregat zum Einsatz bei chirurgischen Eingriffen gelöst, mit einer eine Längsachse definierenden länglichen Ausdehnung, das Zugspindelaggregat umfassend:
- ein Bedienende zum Bedienen des Zugspindelaggregats;
- ein Anschlussende zum Anschließen eines chirurgischen Zubehörs, wie beispielsweise einer Patientenfußhalterung, an das Zugspindelaggregat;
- einen Gewindespindeltrieb mit
   a) einer sich entlang der Längsachse erstreckenden Spindel mit einem Außengewinde; und
   b) einer auf der Spindel sitzenden Spindelmutter mit einem Innengewinde, welches mit dem Außengewinde in Eingriff steht;
- einen durch eine Relativdrehung zwischen der Spindel und der Spindelmutter entlang der Längsachse verfahrbaren Schlitten;
- eine am Bedienende befindliche Antriebseinrichtung zum drehenden Antreiben des Gewindespindeltriebs; und
- ein Getriebe, das zwischen die Antriebseinrichtung und den Gewindespindeltrieb geschaltet ist, wobei das Getriebe dazu eingerichtet ist, eine Drehbewegung der Antriebseinrichtung ins Schnelle übersetzt an den Gewindespindeltrieb auszugeben.

Durch das ins Schnelle übersetzende Getriebe lässt sich das Zugspindelaggregat schnell verfahren. Somit kann der Patientenfuß mit relativ wenigen Umdrehungen der Antriebseinrichtung schnell und effizient an einer gewünschten Stelle positioniert werden.

Vorzugsweise weist das offenbarungsgemäße Zugspindelaggregat mindestens eines der folgenden Merkmale auf:
- eine Hemmvorrichtung zur Hemmung der Relativdrehung zwischen der Spindel und der Spindelmutter, wenn auf das Anschlussende eine Zugkraft entlang der Längsachse ausgeübt wird;
- ein den Gewindespindeltrieb aufnehmendes Gehäuse, wobei die Hemmvorrichtung ein Reibungspaar aufweist, wobei das Reibungspaar aus einer am Gehäuse befindlichen ersten Anlagefläche und einer am Gewindespindeltrieb befindlichen, der ersten Anlagefläche gegenüberliegenden zweiten Anlagefläche besteht, wobei, wenn auf das Anschlussende eine Zugkraft entlang der Längsachse ausgeübt wird, die zweite Anlagefläche sich derart auf der ersten Anlagefläche abstützt, dass ein Reibwiderstand besteht, der eine selbsttätige Relativdrehung zwischen der Spindel und der Spindelmutter infolge der Zugkraft unterbindet;
- die erste und die zweite Anlagefläche erstrecken sich im Wesentlichen senkrecht zur Längsachse;
- die Spindel ist ortsfest und drehbar im Gehäuse angeordnet und die Spindelmutter drehfest mit dem Schlitten verbunden, und wobei die zweite Anlagefläche an der Spindel angeordnet ist;
- das Gehäuse umfasst eine Spindellagerhülse, an welcher die erste Anlagefläche ausgebildet ist, und wobei die Spindel eine drehfest mit ihr verbundene Anlagescheibe aufweist, an welcher die zweite Anlagefläche ausgebildet ist;
- das Getriebe umfasst ein Planetengetriebe;
- das Getriebe ist derart übersetzt, dass eine Umdrehung der Antriebseinrichtung mindestens zwei Umdrehungen des Gewindespindeltriebs entspricht, vorzugsweise derart, dass eine Umdrehung der Antriebseinrichtung 3,5 Umdrehungen des Gewindespindeltriebs entspricht;
- ein Durchmesser der Spindel (32) beträgt weniger als 20mm, insbesondere weniger als 15mm;
- ein Durchmesser der Spindel (32) beträgt 12mm und eine Steigung der Spindel 3mm.

Vorzugsweise kann das Zugspindelaggregat eine zusätzliche Hemmvorrichtung aufweisen. Eine solche zusätzliche Hemmvorrichtung garantiert eine Selbsthemmung des Zugspindelaggregats. Dadurch muss die Selbsthemmung nicht mehr ausschließlich durch die genaue Auswahl der Dimensionierung des Außengewindes der Spindel sichergestellt werden. Dementsprechend kann das Zugspindelaggregat mit einer handelsüblichen Standardspindel versehen sein, die nicht unter allen denkbaren Betriebsbedingungen 100% selbsthemmend sein muss. Die zuverlässige Selbsthemmung übernimmt die genau dimensionierte Hemmvorrichtung.

Dabei kann die Hemmvorrichtung eine Reibkraft erzeugen, die proportional zur am Zugspindelaggregat anliegenden Last ist. Somit wird bei erhöhter Zugkraft auf den Schlitten eine erhöhte Reibkraft durch die Hemmvorrichtung erzielt, sodass eine Selbsthemmung auch unter hoher Last gegeben ist. Die Hemmwirkung der Hemmvorrichtung addiert sich zu der Hemmwirkung des Außengewindes der Spindel.

Gemäß einiger Ausführungsformen kann das Gehäuse eine Spindellagerhülse umfassen, wobei eine mit der Spindel drehfest verbundene Anlagescheibe an der Spindellagerhülse anliegt, so dass die Anlagescheibe zusammen mit einer Anlagefläche der Spindellagerhülse die Hemmvorrichtung bildet. Dadurch kann das Gehäuse kompakt aufgebaut sein. Durch Veränderung der Fläche der Reibflächen zwischen der Anlagescheibe und der Anlagefläche lässt sich die erzeugte Hemmwirkung beeinflussen. Gemäß einiger Ausführungsformen kann das Getriebe ein Planetengetriebe umfassen. Damit kann ein kompakter Aufbau einer Getriebeeinheit erzielt werden, und es kann sichergestellt werden, dass die Drehachse der Antriebseinrichtung mit der Drehachse des Gewindespindeltriebs zusammenfällt.

Das Getriebe kann derart übersetzt sein, dass eine Umdrehung der Antriebseinrichtung mindestens zwei Umdrehungen des Gewindespindeltriebs entspricht. Vorzugsweise ist dabei das Getriebe derart übersetzt, dass eine Umdrehung der Antriebseinrichtung 3,5 Umdrehungen des Gewindespindeltriebs entspricht.

Gemäß einigen Ausführungsformen kann der Gewindespindeltrieb eine Trapezspindel aufweisen. Ein Durchmesser der Spindel kann beispielsweise weniger als 20mm, insbesondere weniger als 15mm betragen. Dadurch kann das Zugspindelaggregat kompakt aufgebaut werden, und es ist nicht notwendig, beispielsweise ein Stator-Rohr mit einem hohen Innendurchmesser vorzusehen, in dem die Spindel drehbar aufgenommen ist.

Als beispielhafte Ausführungsform kann dabei der Durchmesser der Spindel 12mm und die Steigung der Spindel 3 mm betragen. Eine derartige Spindel ist nahezu selbsthemmend. In Kombination mit einem Getriebe mit einer Übersetzung von 3,5 Umdrehungen des Gewindespindeltriebs pro Umdrehung der Antriebseinrichtung kann dann ein Verstellweg von 10,5 mm pro Umdrehung der Antriebseinrichtung erzielt werden.

Die vorliegende Offenbarung betrifft ebenfalls einen Extensionsholm für einen Operationstisch, umfassend:
- einen Holm, welcher am Operationstisch anbringbar ist;
- eine verschiebbare Strebe, welche entlang einer Längsachse des Holms verschiebbar ist, und welche eine Halterung für einen Fuß eines Patienten trägt; und
- ein wie oben definiertes Zugspindelaggregat, um die Halterung für den Fuß des Patienten relativ zu einer Position der Strebe in einer Richtung im Wesentlichen parallel zu einer Längsachse des Zugspindelaggregats zu verstellen.

Gemäß einem weiteren Aspekt wird ein Extensionsholm für einen Operationstisch bereitgestellt, welcher einen Holm, welcher am Operationstisch anbringbar ist, und eine verschiebbare Strebe umfasst, welche entlang einer Längsachse des Holms verschiebbar ist, und welche eine Halterung für einen Fuß eines Patienten trägt. Weiterhin umfasst der Extensionsholm ein Zugspindelaggregat wie voranstehend beschrieben, um die Halterung für den Fuß des Patienten relativ zu einer Position der Strebe in einer Richtung im Wesentlichen parallel zu einer Längsachse des Zugspindelaggregats zu verstellen.

### Kurze Beschreibung der Zeichnungen

Beispielhafte Ausführungsformen der vorliegenden Offenbarung werden nachstehend unter Bezugnahme auf die angehängten Zeichnungen beschrieben, in welchen gleiche Bezugszeichen jeweils gleiche oder einander entsprechende Elemente bezeichnen.
- Fig. 1: zeigt eine Darstellung eines Operationstisches mit einem Extensionsholm, bei dem ein Zugspindelaggregat gemäß einer Ausführungsform der vorliegenden Offenbarung im Einsatz ist.
- Fig. 2: zeigt ein Zugspindelaggregat gemäß einer Ausführungsform der vorliegenden Offenbarung.
- Fig. 3: ist ein Teillängsschnitt des Zugspindelaggregats aus Fig. 2 gemäß den Pfeilen II-II.
- Fig. 4: zeigt einige Bauteile des Zugspindelaggregats aus Fig. 2.
- Fig. 5: zeigt ein Getriebe, das bei dem Zugspindelaggregat aus Fig. 2 verwendet werden kann.
- Fig. 6: zeigt einige Bauteile zur Lagerung der Spindel bei dem Zugspindelaggregat aus Fig. 2.

In der folgenden Beschreibung werden unter Bezugnahme auf die Zeichnungen beispielhafte Ausführungsformen der vorliegenden Offenbarung beschrieben. Die Zeichnungen sind dabei nicht notwendigerweise maßstabsgetreu, sondern sollen die jeweiligen Merkmale lediglich schematisch illustrieren.

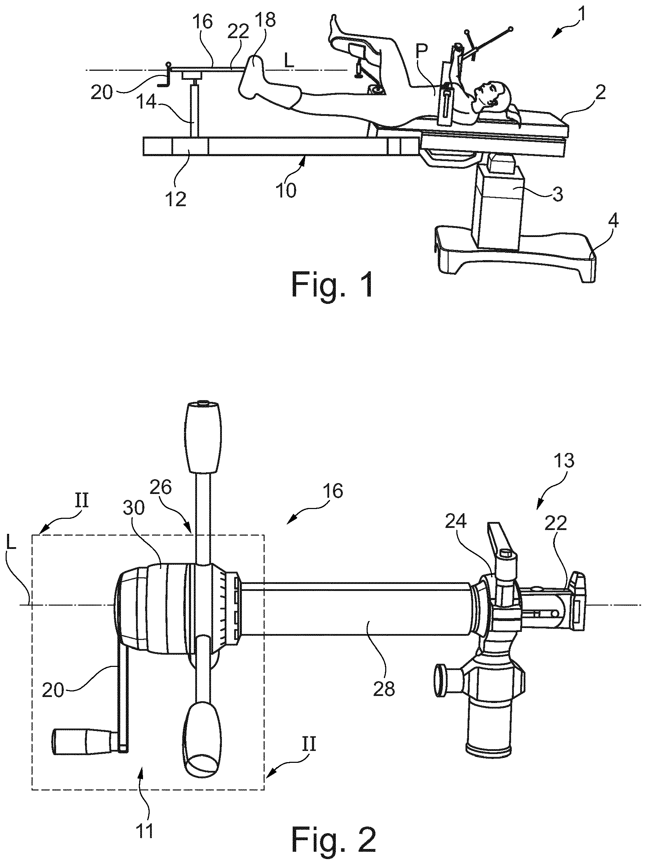

Wie in Fig. 1 dargestellt, umfasst ein Operationstisch 1 eine Patientenlagerfläche 2, eine Säule 3 und einen Fuß 4. Zur Durchführung von orthopädischen Operationen kann ein Extensionsholm 10 verwendet werden, mittels dessen das Bein eines Patienten P in einer gewünschten Position fixiert werden kann. Hierzu kann an einem Holm 12 des Extensionsholms 10 eine verschiebbare Strebe 14 angebracht sein, die wiederum ein Zugspindelaggregat 16 trägt, das mit einer Halterung 18 für einen Patientenfuß verbunden ist.

Wenn die verschiebbare Strebe 14 in einer geeigneten Position am Holm 12 fixiert ist, kann somit über das Zugspindelaggregat 16 Zug auf den Patientenfuß in einer Richtung entlang der Längsachse L des Zugspindelaggregats 16 ausgeübt werden, um beispielsweise ein Hüftgelenk des Patienten P auseinander zu ziehen. Der Verfahrweg des Zugspindelaggregats 16 kann hierbei bis zu 20cm betragen, um Zug auf die eingespannte Extremität auszuüben.

Dazu wird allgemein ein Schlitten 22 des Zugspindelaggregats 16 translatorisch verfahren. Der Schlitten 22 muss dabei unter Zuglast an der eingestellten Position stehenbleiben, d.h. das Zugspindelaggregat muss selbsthemmend ausgeführt sein.

Fig. 2 zeigt eine Darstellung des Zugspindelaggregats 16 aus Fig. 1 gemäß einer Ausführungsform der vorliegenden Offenbarung.

Das Zugspindelaggregat 16 hat ein Bedienende 11, mit welchem es der Anwender bedienen kann. Es hat ebenfalls ein Anschlussende 13 zum Anschließen eines chirurgischen Zubehörs.

Das Zugspindelaggregat 16 ist über eine Aufnahmehülse 24 mit der verschiebbaren Strebe 14 des Extensionsholms 10 (siehe Fig. 1) verbindbar. Am Anschlussende 13 kann an dem Schlitten 22 des Zugspindelaggregats 16 die Halterung 18 für einen Patientenfuß befestigt werden (siehe Fig. 1).

Das Zugspindelaggregat 16 umfasst außerdem eine Antriebseinrichtung in Form einer Handkurbel 20, mittels derer der Schlitten 22 in Richtung der Längsachse L des Zugspindelaggregats 16 bewegbar ist.

Zusätzlich zur Verstellung in Längsrichtung kann am Zugspindelaggregat 16 auch ein Rastmechanismus 26 vorgesehen sein, welcher eine Drehung des Schlittens 22 und der daran befestigten Fußhalterung 18 um die Längsachse L ermöglicht. Der Rastmechanismus 26 wird in der vorliegenden Beschreibung nicht detailliert ausgeführt, und ist unabhängig von der hier beschriebenen Verstellung des Schlittens 22 in Längsrichtung.

Das Zugspindelaggregat 16 umfasst weiterhin ein Statorrohr 28, das einen Teil eines Gehäuses des Zugspindelaggregats 16 bildet und innerhalb dessen ein Gewindespindeltrieb drehbar angeordnet ist, wie nachfolgend im Einzelnen beschrieben werden wird. Weiterhin ist ein Getriebe 30 zwischen der Handkurbel 20 und dem Gewindespindeltrieb angeordnet. Vorzugsweise wird hierbei ein Planetengetriebe verwendet, so dass die Antriebsachse und die Abtriebsachse des Getriebes 30 beide auf der Längsachse L des Zugspindelaggregats liegen.

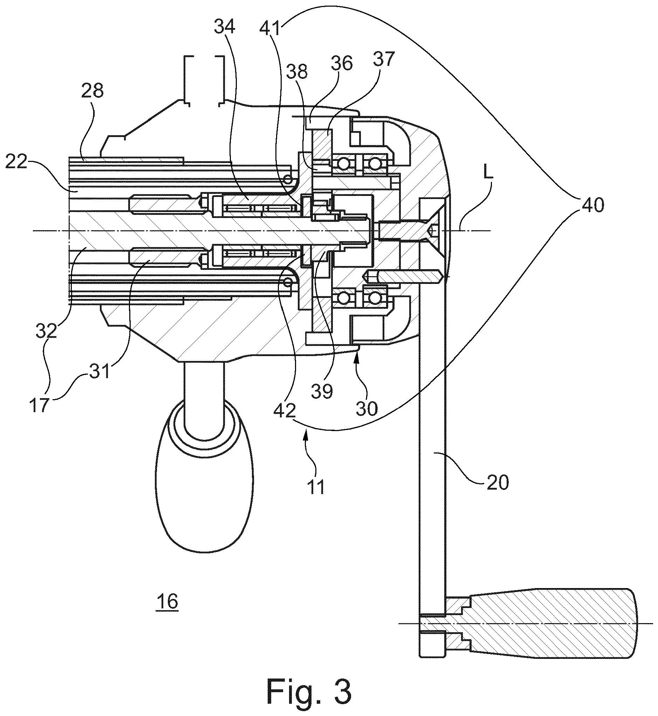

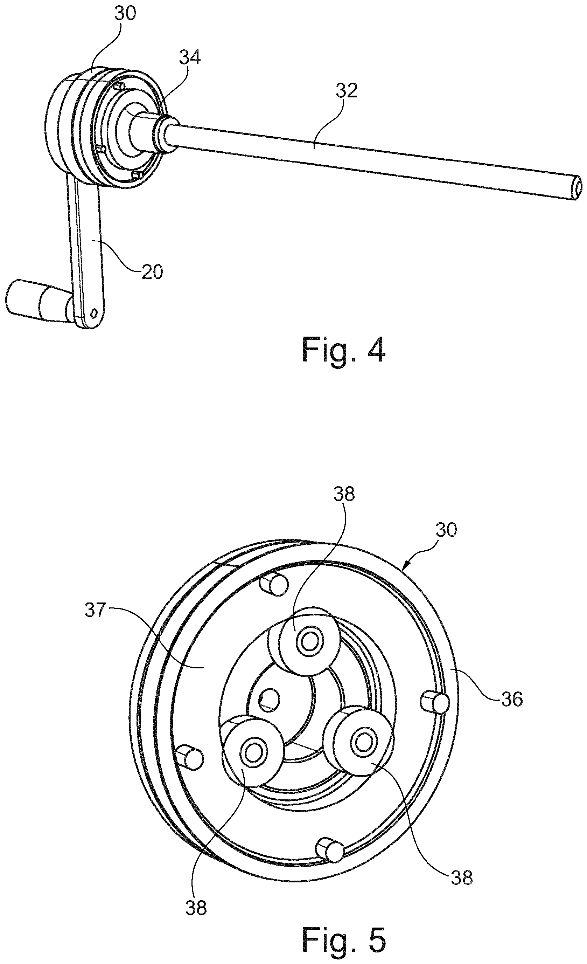

Die Fig. 3 und 4 zeigen den Innenaufbau des Zugspindelaggregats 16 aus Fig. 2. Man erkennt den Gewindespindeltrieb 17. Dieser umfasst eine sich entlang der Längsachse L erstreckende Spindel 32, die im Statorrohr 28 drehbar aufgenommen ist. Die Spindel 32 ist dabei in einer Spindellagerhülse 34 gelagert. Bei der vorliegenden Ausführungsform ist die Spindel 32 eine Trapezspindel mit einem Durchmesser von 12mm und einer Steigung von 3mm. Damit hat die Spindel 32 ein Außengewinde, welches unter den meisten Betriebsbedingungen selbsthemmend ist.

Es ist möglich, andere Arten von Gewindespindeln, wie beispielsweise Spindeln mit Spitzgewinde oder Sägengewinde, zu verwenden.

Der Gewindespindeltrieb 17 besitzt auch eine auf der Spindel 32 sitzende Spindelmutter 31 mit einem Innengewinde, welches mit dem Außengewinde der Spindel in Eingriff steht.

Der Schlitten 22 ist durch eine Relativdrehung zwischen der Spindel 32 und der Spindelmutter 31 entlang der Längsachse L verfahrbar.

Im vorliegenden Beispiel ist die Spindel 32 ortsfest und drehbar im Statorrohr 28 angeordnet und die Spindelmutter 31 drehfest und axial verschieblich mit dem Schlitten 22 verbunden. Zum Verfahren des Schlittens 22 wird die Spindel 32 gedreht, sodass sich die Spindelmutter 31 linear auf der Spindel 32 verschiebt.

Es ist aber auch möglich, den Gewindespindeltrieb kinematisch umgekehrt auszuführen, sodass die Spindelmutter ortsfest und drehbar angeordnet ist, und die Spindel drehfest mit dem Schlitten verbunden ist. In diesem Fall verfährt die Spindel gemeinsam linear mit dem Schlitten relativ zur Spindelmutter.

Bevorzugt besitzt das Zugspindelaggregat 16 eine zusätzliche Hemmvorrichtung 40 zur Hemmung der Relativdrehung zwischen der Spindel 32 und der Spindelmutter 31, wenn auf das Anschlussende 13 eine Zugkraft entlang der Längsachse L ausgeübt wird. Diese Hemmvorrichtung 40 weist ein Reibungspaar 41, 42 auf, wobei das Reibungspaar aus einer am Gehäuse, nämlich an der Spindellagerhülse 34, befindlichen ersten Anlagefläche 41 und einer am Gewindespindeltrieb 17 befindlichen, der ersten Anlagefläche gegenüberliegenden zweiten Anlagefläche 42 besteht. Wenn dann auf das Anschlussende 13 eine Zugkraft entlang der Längsachse L ausgeübt wird, stützt sich die zweite Anlagefläche 42 derart auf der ersten Anlagefläche 41 ab, dass ein Reibwiderstand besteht, der eine selbsttätige Relativdrehung zwischen der Spindel 32 und der Spindelmutter 31 infolge der Zugkraft unterbindet. Dieser Reibwiderstand wächst proportional zur anliegenden Axiallast an. Aus der Fig. 3 ist ersichtlich, dass die erste und die zweite Anlagefläche 41, 42 sich im Wesentlichen senkrecht zur Längsachse L erstrecken. Die Flächen der Anlageflächen sind so bemessen, dass die Selbsthemmung mit ausreichender Sicherheit hergestellt wird.

Die zweite Anlagefläche 42 ist im vorliegenden Fall in Form einer drehfest mit der Spindel 32 verbundenen Anlagescheibe verwirklicht.

Um mit der Handkurbel 20 den geforderten hohen Verstellweg pro Kurbelumdrehung zu generieren, ist bei der gezeigten Ausführungsform zwischen dieser und der Spindel 32 ein Planetengetriebe 30 angeordnet, das die Handkurbelumdrehung ins Schnelle übersetzt, so dass die Spindel 32 bei einer Handkurbelumdrehung z. B. etwa 3,5 Umdrehungen ausführt und somit der geforderte Verstellweg des Schlittens 22 pro Handkurbelumdrehung erreicht wird.

Ein Beispiel für ein derartiges Planetengetriebe 30 ist in Fig. 5 gezeigt. Hierbei ist ein Hohlrad 36 orstfest im Statorrohr 28 verschraubt, und ein Planetenträger 37 ist mit der Handkurbel 20 verbunden. Der Planetenträger 37 treibt bei dem gezeigten Beispiel drei Planetenräder 38 an, welche wiederum ein (in Fig. 3 gezeigtes) im Zentrum liegendes Sonnenrad 39 antreiben, das mit der Spindel 32 verbunden ist. Die Drehachse des Planetenträgers 37, und somit die Drehachse der Handkurbel 20, fällt hierbei mit der Drehachse des Sonnenrads, und somit der Drehachse der Spindel 32, zusammen.

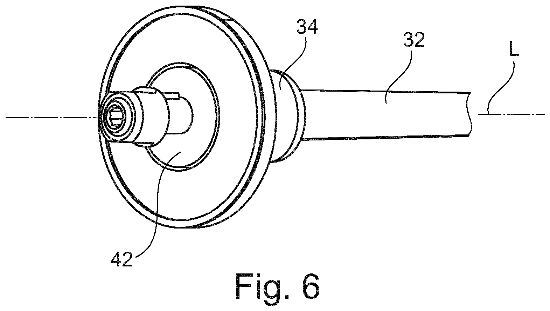

Fig. 6 zeigt die Lagerung der Spindel 32 in der Spindellagerhülse 34. Dabei ist die Spindellagerhülse 34 fest am Statorrohr 28 oder an einem anderen Teil des Gehäuses des Zugspindelaggregats 16 befestigt.

Die Anlagescheibe 42 ist an der Spindel 32 befestigt und ist somit relativ zu der Spindellagerhülse 34 drehbar. Die Spindel 32 stützt sich dabei mittels der Anlagescheibe 42 in axialer Richtung entlang der Längsachse L an der Anlagefläche 41 an der Spindellagerhülse 34 ab. Die Reibkraft zwischen der Anlagescheibe 42 und der Anlagefläche 41 der Spindellagerhülse 34 ist proportional zur an der Spindel 32 anliegenden axialen Last. Der Durchmesser der Anlagescheibe 42 ist dabei ausreichend groß gewählt, sodass die Selbsthemmung des Zugspindelaggregats 16 bei allen im Betrieb zu erwartenden Zugkräften mit der erforderlichen Sicherheit erreicht wird.

Die vorliegende Offenbarung stellt somit ein Zugspindelaggregat bereit, das selbsthemmend ist und das über einen großen Verstellweg pro Kurbelumdrehung verfügt.

Das voranstehend beschriebene Zugspindelaggregat ermöglicht eine gute Bedienergonomie, da ein Benutzer einerseits mit relativ wenigen Umdrehungen der Handkurbel einen relativ großen Verstellweg erreichen kann, und andererseits eine Selbsthemmung der Zugspindel auch bei hohen Zugkräften sichergestellt werden kann.

Der für die Bedienung angemessen große Verstellweg pro Handkurbelumdrehung wird durch den Einsatz des ins Schnelle übersetzende Getriebe erreicht.

Wenn auch noch die zusätzliche Hemmvorrichtung vorgesehen ist, muss bei der Auswahl der Spindel des Gewindespindeltriebs nicht mehr darauf geachtet werden, dass diese stets selbsthemmend ist. Dadurch können Spindeln mit größerer Steigung verwendet werden. Eine größere Spindelsteigung hat den Vorteil, dass das ins Schnelle übersetzende Getriebe zur Erzielung des angemessen großen Verstellwegs ein kleineres Übersetzungsverhältnis haben kann. Es kann also ein kompaktes und einfach aufgebautes Getriebe verwendet werden.

Im Übrigen kann das Zugspindelaggregat der vorliegenden Offenbarung auch noch die folgenden Merkmale aufweisen, welche die Selbsthemmung des Zugspindelaggregats ermöglichen:
- eine Spindel 32 mit einem Durchmesser von 25mm oder weniger, 20mm oder weniger, oder 15 mm oder weniger;
- Die Spindel hat Gewindesteigungen von 2mm oder mehr, 2,5mm oder mehr, oder 3mm oder mehr;
- Die Gewindespindel und die Spindelmutter haben derartige Abmessungen, Durchmesser und Gewindesteigungen, dass das Zugspindelaggregat nicht alleine dank deren Gewinde vollkommen selbsthemmend ist, ohne die zusätzliche Hemmvorrichtung 40. Zum Beispiel ist das Zugspindelaggregat ohne die Hemmvorrichtung 40 nicht vollkommen selbsthemmend, wenn die Gewindespindel 32 Axialkräften ausgesetzt ist, die man beim Einsatz zu erwarten hat;
- Die Hemmvorrichtung hat eine erste Fläche 41 und eine zweite Fläche 42 verschiedener Formen und Anordnungen;
- Die zwei Flächen 41 und 42 sind einander gegenüber liegend positioniert, derart, dass ein axiales Ziehen an der Spindel 32 sie zusammen drückt und Reibung zwischen den zwei Flächen 41 und 42 erzeugt. Die Reibung hemmt eine Relativdrehung zwischen den Flächen 41, 42. Die Reibung ist vorzugsweise ausreichend, um eine Drehung und eine Axialbewegung entlang des Spindelgewindes in Antwort auf ein axiales Ziehen an der Spindel 32 zu verhindern;
- Der kombinierte Widerstand der Gewindespindel 32 und der Hemmvorrichtung 40 ist notwendig, um eine Drehung und eine Axialbewegung entlang des Spindelgewindes in Antwort auf ein axiales Ziehen an der Spindel 32 zu verhindern

## Patentansprüche

1. Selbsthemmendes Zugspindelaggregat (16) zum Einsatz bei chirurgischen Eingriffen, mit einer eine Längsachse (L) definierenden länglichen Ausdehnung, das Zugspindelaggregat umfassend:
- ein Bedienende (11) zum Bedienen des Zugspindelaggregats;
- ein Anschlussende (13) zum Anschließen eines chirurgischen Zubehörs an das Zugspindelaggregat;
- einen Gewindespindeltrieb (17) mit
a) einer sich entlang der Längsachse (L) erstreckenden Spindel (32) mit einem Außengewinde; und
b) einer auf der Spindel (32) sitzenden Spindelmutter (31) mit einem Innengewinde, welches mit dem Außengewinde in Eingriff steht;
- einen durch eine Relativdrehung zwischen der Spindel und der Spindelmutter entlang der Längsachse verfahrbaren Schlitten (22);
- eine am Bedienende (11) befindliche Antriebseinrichtung (20) zum drehenden Antreiben des Gewindespindeltriebs;
**dadurch gekennzeichnet, dass**
das Zugspindelaggregat (16) ferner ein Getriebe (30) umfasst, das zwischen die Antriebseinrichtung (20) und den Gewindespindeltrieb (17) geschaltet ist, wobei das Getriebe dazu eingerichtet ist, eine Drehbewegung der Antriebseinrichtung (20) ins Schnelle übersetzt an den Gewindespindeltrieb (17) auszugeben, wobei sich das Zugspindelaggregat (16) durch das ins Schnelle übersetzende Getriebe (30) schnell verfahren lässt.

2. Zugspindelaggregat (16) nach Anspruch 1, weiterhin mit einer Hemmvorrichtung (40) zur Hemmung der Relativdrehung zwischen der Spindel und der Spindelmutter, wenn auf das Anschlussende (13) eine Zugkraft entlang der Längsachse ausgeübt wird.

3. Zugspindelaggregat (16) nach Anspruch 2, ferner umfassend ein den Gewindespindeltrieb aufnehmendes Gehäuse, wobei die Hemmvorrichtung ein Reibungspaar aufweist, wobei das Reibungspaar (41, 42) aus einer am Gehäuse befindlichen ersten Anlagefläche (41) und einer am Gewindespindeltrieb befindlichen, der ersten Anlagefläche gegenüberliegenden zweiten Anlagefläche (42) besteht, wobei, wenn auf das Anschlussende (13) eine Zugkraft entlang der Längsachse (L) ausgeübt wird, die zweite Anlagefläche sich derart auf der ersten Anlagefläche abstützt, dass ein Reibwiderstand hervorgerufen wird, der eine selbsttätige Relativdrehung zwischen der Spindel (32) und der Spindelmutter (31) infolge der Zugkraft unterbindet.

4. Zugspindelaggregat (16) nach Anspruch 3, wobei die erste und die zweite Anlagefläche sich im Wesentlichen senkrecht zur Längsachse erstrecken.

5. Zugspindelaggregat (16) nach Anspruch 3 oder 4, wobei die Spindel ortsfest und drehbar im Gehäuse angeordnet ist und die Spindelmutter drehfest mit dem Schlitten verbunden ist, und wobei die zweite Anlagefläche an der Spindel angeordnet ist.

6. Zugspindelaggregat (16) nach Anspruch 5, wobei das Gehäuse eine Spindellagerhülse (34) umfasst, an welcher die erste Anlagefläche ausgebildet ist, und wobei die Spindel (32) eine drehfest mit ihr verbundene Anlagescheibe (42) aufweist, an welcher die zweite Anlagefläche ausgebildet ist.

7. Zugspindelaggregat (16) nach einem der Ansprüche 1 bis 6, wobei das Getriebe (30) ein Planetengetriebe umfasst.

8. Zugspindelaggregat (16) nach einem der Ansprüche 1 bis 7, wobei das Getriebe (30) derart übersetzt ist, dass eine Umdrehung der Antriebseinrichtung (20) mindestens zwei Umdrehungen des Gewindespindeltriebs entspricht, vorzugsweise derart, dass eine Umdrehung der Antriebseinrichtung (20) 3,5 Umdrehungen des Gewindespindeltriebs entspricht.

9. Zugspindelaggregat (16) nach einem der Ansprüche 1 bis 8, wobei ein Durchmesser der Spindel (32) weniger als 20mm, insbesondere weniger als 15mm beträgt.

10. Zugspindelaggregat (16) nach Anspruch 9, wobei ein Durchmesser der Spindel (32) 12mm und eine Steigung der Spindel 3mm beträgt.

11. Extensionsholm (10) für einen Operationstisch (1), umfassend:
- einen Holm (12), welcher am Operationstisch (1) anbringbar ist;
- eine verschiebbare Strebe (14), welche entlang einer Längsachse des Holms (12) verschiebbar ist, und welche eine Halterung (18) für einen Fuß eines Patienten trägt;
**dadurch gekennzeichnet, dass**
der Extensionsholm (10) für den Operationstisch (1) ferner ein Zugspindelaggregat (16) nach einem der Ansprüche 1 bis 10 umfasst, um die Halterung (18) für den Fuß des Patienten relativ zu einer Position der Strebe (14) in einer Richtung im Wesentlichen parallel zu einer Längsachse (L) des Zugspindelaggregats (16) zu verstellen.

## Claims

1. A self-locking traction unit (16) for use in surgical procedures, with an elongate extension defining a longitudinal axis (L), the traction unit comprising:
- an operator (11) for operating the traction unit;
- a connecting end (13) for connecting a surgical accessory to the traction unit;
- a threaded spindle drive (17) with
a) a spindle (32) with an external thread extending along the longitudinal axis (L); and
b) a spindle nut (31) with an internal thread seated on the spindle (32) which engages with the external thread;
- a carriage (22) which can be displaced along the longitudinal axis through a relative rotation between the spindle and the spindle nut;
- a drive device (20) located at the operating end (11) for rotatingly driving the threaded spindle drive;
**characterized in that**
the traction unit (16) further comprises a gear mechanism (30) which is connected between the drive device (20) and the threaded spindle drive (17), wherein the gear mechanism is designed to output a rotary movement of the drive device (20) to the threaded spindle drive (17) in a stepped-up manner, and wherein the traction unit (16) can be moved quickly by means of the step-up gear mechanism (30).

2. The traction unit (16) according to claim 1, further comprising a locking device (40) for locking the relative rotation between the spindle and the spindle nut when a pulling force is exerted on the connecting end (13) along the longitudinal axis.

3. The traction unit (16) according to claim 2, further comprising a housing receiving the threaded spindle drive, wherein the locking device has a friction pair, wherein the friction pair (41, 42) consists of a first contact surface (41) located on the housing and a second contact surface (42) located on the threaded spindle drive and opposite the first contact surface, wherein, when a tensile force is exerted on the connecting end (13) along the longitudinal axis (L), the second contact surface is supported on the first contact surface in such a way that a frictional resistance is caused which prevents an automatic relative rotation between the spindle (32) and the spindle nut (31) as a result of the tensile force.

4. The traction unit (16) according to claim 3, wherein the first and second contact surfaces extend substantially perpendicular to the longitudinal axis.

5. The traction unit (16) according to claim 3 or 4, wherein the spindle is arranged so as to be stationary and rotatable in the housing and the spindle nut is connected in a torque-proof manner to the carriage, and wherein the second contact surface is arranged on the spindle.

6. The traction unit (16) according to claim 5, wherein the housing comprises a spindle bearing sleeve (34) on which the first contact surface is formed, and wherein the spindle (32) has a contact disc (42) which is rotationally connected thereto and on which the second contact surface is formed.

7. The traction unit (1) according to any one of claims 1 to 6, wherein the gear mechanism (30) comprises a planetary gear.

8. The traction unit (16) according to any one of claims 1 to 7, wherein the gear mechanism (30) is geared such that one revolution of the drive device (20) corresponds to at least two revolutions of the threaded spindle drive, preferably such that one revolution of the drive device (20) corresponds to 3.5 revolutions of the threaded spindle drive.

9. The traction unit (16) according to any one of claims 1 to 8, wherein a diameter of the spindle (32) is less than 20 mm, in particular less than 15 mm.

10. The traction unit (16) according to claim 9, wherein a diameter of the spindle (32) is 12 mm and a pitch of the spindle is 3 mm.

11. An extension bar (10) for an operating table (1), comprising:
- a bar (12) which can be attached to the operating table (1);
- a displaceable strut (14) which can be displaced along a longitudinal axis of the bar (12) and which carries a holder (18) for a patient's foot;
**characterized in that**
the extension bar (10) for the operating table (1) further comprises a traction unit (16) according to any one of claims 1 to 10 in order to adjust the holder (18) for the patient's foot relative to a position of the strut (14) in a direction substantially parallel to a longitudinal axis (L) of the traction unit (16).

## Revendications

1. Appareil à tige de traction (16) autobloquant, destiné à être utilisé lors d'interventions chirurgicales, pourvu d'une extension allongée définissant un axe longitudinal (L), l'appareil à tige de traction comprenant :
- une extrémité de service (11) pour faire fonctionner l'appareil à tige de traction ;
- une extrémité de raccord (13) pour raccorder un accessoire chirurgical à l'appareil à tige de traction ;
- un entraînement à tige filetée (17) présentant
a) une tige (32) s'étendant le long de l'axe longitudinal (L) et pourvue d'un filetage externe ; et
b) un écrou de tige (31) placé sur la tige (32), pourvu d'un filetage interne, qui est en prise avec le filetage externe ;
- un coulisseau (22) pouvant être déplacé le long de l'axe longitudinal par une rotation relative entre la tige et l'écrou de tige ;
- un dispositif d'entraînement (20) se trouvant à l'extrémité de service (11) pour l'entraînement en rotation de l'entraînement à tige filetée ;
**caractérisé en ce que**
l'appareil à tige de traction (16) comprend en outre un mécanisme (30) qui est monté entre le dispositif d'entraînement (20) et l'entraînement à tige filetée (17), dans lequel le mécanisme est configuré pour transmettre un mouvement de rotation du dispositif d'entraînement (20) transformé en rapidité à l'entraînement à tige filetée (17), dans lequel l'appareil à tige de traction (16) peut être déplacé rapidement par le mécanisme (30), qui se transforme en rapidité.

2. Appareil à tige de traction (16) selon la revendication 1, pourvu en outre d'un dispositif d'arrêt (40) pour empêcher la rotation relative entre la tige et l'écrou de tige, lorsqu'une force de traction est appliquée à l'extrémité de raccord (13) le long de l'axe longitudinal.

3. Appareil à tige de traction (16) selon la revendication 2, comprenant en outre un boîtier recevant l'entraînement à tige filetée, dans lequel le dispositif d'arrêt présente une paire de frottement, dans lequel la paire de frottement (41, 42) est constituée d'une première surface de contact (41) se trouvant sur le boîtier et d'une seconde surface de contact (42) opposée à la première surface de contact, se trouvant sur l'entraînement à tige filetée, dans lequel lorsqu'une force de traction est appliquée à l'extrémité de raccord (13) le long de l'axe longitudinal (L), la seconde surface de contact s'appuie sur la première surface de contact de sorte qu'une résistance de frottement est générée, ce qui empêche une rotation relative automatique entre la tige (32) et l'écrou de tige (31) en raison de la force de traction.

4. Appareil à tige de traction (16) selon la revendication 3, dans lequel les première et seconde surfaces de contact s'étendent sensiblement perpendiculairement à l'axe longitudinal.

5. Appareil à tige de traction (16) selon la revendication 3 ou 4, dans lequel la tige est disposée de manière fixe et rotative dans le boîtier et l'écrou de tige est relié au coulisseau de manière fixe en rotation, et dans lequel la seconde surface de contact est disposée sur la tige.

6. Appareil à tige de traction (16) selon la revendication 5, dans lequel le boîtier comprend un manchon de palier de tige (34) sur lequel la première surface de contact est formée, et dans lequel la tige (32) présente un disque de contact (42) qui lui est relié de manière fixe en rotation et sur lequel est formée la seconde surface de contact.

7. Appareil à tige de traction (16) selon l'une des revendications 1 à 6, dans lequel le mécanisme (30) comprend un engrenage planétaire.

8. Appareil à tige de traction (16) selon l'une des revendications 1 à 7, dans lequel le mécanisme (30) est adapté de sorte qu'un tour du dispositif d'entraînement (20) correspond à au moins deux tours de l'entraînement à tige filetée, de préférence de sorte qu'un tour du dispositif d'entraînement (20) correspond à 3,5 tours de l'entraînement à tige filetée.

9. Appareil à tige de traction (16) selon l'une des revendications 1 à 8, dans lequel un diamètre de la tige (32) est inférieur à 20 mm, en particulier inférieur à 15 mm.

10. Appareil à tige de traction (16) selon la revendication 9, dans lequel un diamètre de la tige (32) est de 12 mm et le pas de la tige est de 3 mm.

11. Barre d'extension (10) pour table d'opération (1), comprenant :
- une barre (12), qui peut être fixée à la table d'opération (1) ;
- une entretoise pouvant être déplacée (14), qui peut être déplacée le long d'un axe longitudinal de la barre (12) et qui porte un support (18) pour le pied d'un patient ;
**caractérisée en ce que**
la barre d'extension (10) pour la table d'opération (1) comprend en outre un appareil à tige de traction (16) selon l'une des revendications 1 à 10, pour régler le support (18) pour le pied du patient par rapport à une position de l'entretoise (14) dans une direction sensiblement parallèle à un axe longitudinal (L) de l'appareil à tige de traction (16).
